# EUROPEAN PATENT APPLICATION

(11) **EP 4 813 998 A2**
(43) Date of publication of application: **30.09.2026**
(21) Application number: 26183079.8
(22) Date of filing: 20.12.2022
(51) Int. Cl.: C07H 19/10

(54) **CHEMICAL SYNTHESIS OF CYTIDINE-5'-MONOPHOSPHO-N-GLYCYL-SIALIC ACID**

(30) Priority: 20.12.2021 US 202163265744 P
(62) Document number(s) of the earlier application(s) in accordance with Art. 76 EPC: 22888633.9 / 4 225 766
(71) Applicant: 89Bio, Inc., San Francisco, CA 94104 (US)
(72) Inventor: JOSEPH, Shaji, Sunnyvalle, CA94087 (US); BODENMÜLLER, Arthur, 4133 Pratteln (CH)
(74) Representative: Sagittarius IP

(57) **Abstract**

Aspects of the present disclosure provide methods for the chemical synthesis of cytidine-5'-monophospho-N-glycyl-sialic acid (GSC).

## Description

### RELATED APPLICATIONS

This application claims priority to, and the benefit of, U.S. provisional Application No. 63/265,744, filed December 20, 2021, the content of which is incorporated herein by reference in its entirety.

### FIELD

The present disclosure relates to the field of chemical synthesis of cytidine-5'-monophospho-N-glycyl-sialic acid.

### SUMMARY

Aspects of the present disclosure provides for a method of synthesis cytidine-5'-monophospho-N-glycyl-sialic acid (GSC).

In some embodiments, the method comprising two or more steps, wherein the first step of the two or more steps comprise benzylation of N-Acetylneuraminic acid. In some embodiments, the first step comprises the steps of synthesizing benzyl (2S,4S,5R)-5-acetamido-2,4-dihydroxy-6-[(1R,2R)-1,2,3-trihydroxypropyl]tetrahydropyran-2-carboxylate. In some embodiments, the two or more steps comprises the step of synthesising (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid or a salt thereof. In some embodiments, the two or more steps comprises the step of synthesizing benzyl (2S,4S,5R)-5-acetamido-2,4-dihydroxy-6-[(1R,2R)-1,2,3-trihydroxypropyl]tetrahydropyran-2-carboxylate, the step of synthesizing benzyl (2R,4S,5R)-5-acetamido-2,4-diacetoxy-6-[(1S,2R)-1,2,3-triacetoxypropyl]tetrahydropyran-2-carboxylate, the step of synthesizing benzyl (2R,4S,5R)-5-acetamido-4-acetoxy-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate, the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium, the step of synthesizing benzyl (2R,4S,5R)-4-acetoxy-2-phenylsulfanyl-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2S,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing [(2R,4S,5R)-4-acetoxy-2-carboxy-6-[(1S,2R)-1,2,3-triacetoxypropyl]-5-[[2-[(2,2,2-trifluoroacetyl)amino]acetyl]aminotetrahydropyran-2-yl] hydrogen phosphate;triethylammonium, the step of synthesizing (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid or salt thereof.

Provided herein is a method of synthesizing cytidine-5'-monophospho-N-glycyl-sialic acid (GSC), the method comprising the step of synthesizing benzyl (2S,4S,5R)-5-acetamido-2,4-dihydroxy-6-[(1R,2R)-1,2,3-trihydroxypropyl]tetrahydropyran-2-carboxylate, the step of synthesizing benzyl (2R,4S,5R)-5-acetamido-2,4-diacetoxy-6-[(1S,2R)-1,2,3-triacetoxypropyl]tetrahydropyran-2-carboxylate, the step of synthesizing benzyl (2R,4S,5R)-5-acetamido-4-acetoxy-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate, the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium, the step of synthesizing benzyl (2R,4S,5R)-4-acetoxy-2-phenylsulfanyl-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2S,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing [(2R,4S,5R)-4-acetoxy-2-carboxy-6-[(1S,2R)-1,2,3-triacetoxypropyl]-5-[[2-[(2,2,2-trifluoroacetyl)amino]acetyl]amino]tetrahydropyran-2-yl] hydrogen phosphate;triethylammonium, the step of synthesizing (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid or salt thereof or any combinations of the foregoing steps. In some emboeidments, the starting material is N-Acetylneuraminic acid.

Provided herein is a method of synthesizing cytidine-5'-monophospho-N-glycyl-sialic acid (GSC) from N-Acetylneuraminic acid, the method comprising one or more of benzylation, acetylation, thiophenol introduction, Boc protection, deacetylation, Boc deprotection, TFA-Gly introduction, thiophenol removal, phosphite introduction, debenzylation, triacetyl-cytidine coupling, acetyl and trifuoroacetamide deprotection reactions.

Provided herein is a process for the preparation of cytidine-5'-monophospho-N-glycyl-sialic acid (GSC) comprising:
a. Benzylation of Neu5AC to obtain Intermediate 1 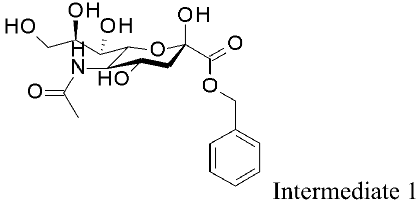
b. Acetylation of Intermediate 1 to obtain intermediate 2 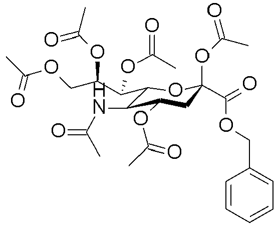 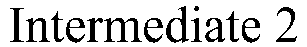
c. Thiophenol introduction of intermediate 2 to obtain intermediate 3 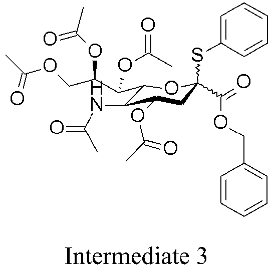
d. Boc protection of intermediate 3 to obtain intermediate 4 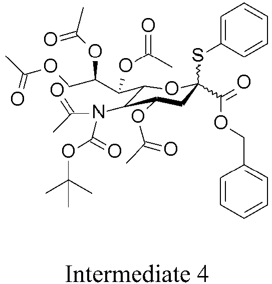
e. Deacetylation of intermediate 4 to obtain intermediate 5 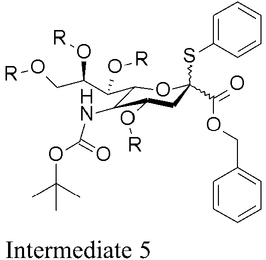
f. Acetylation of intermediate 5 to obtain intermediate 6 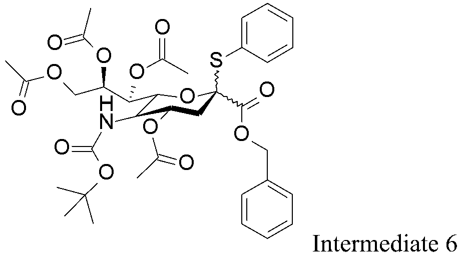
g. Boc deprotection of intermediate 6 to obtain intermediate 7 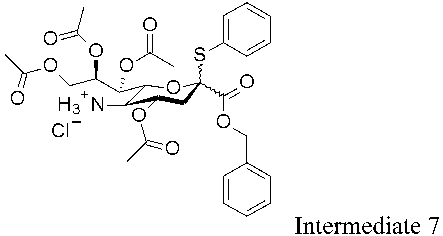
h. TFA-Gly introduction to intermediate 7 to obtain intermediate 8 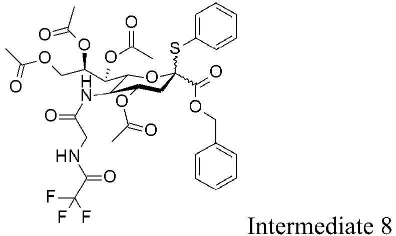
i. Thiophenol removal to intermediate 8 to obtain intermediate 9 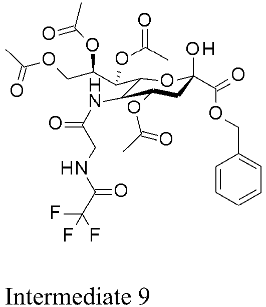
j. Phosphite introduction to intermediate 9 to obain intermediate 10 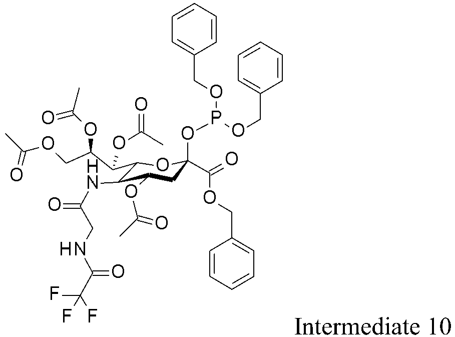
**k.** oxidation of intermediate 10 to obtain intermediate 11 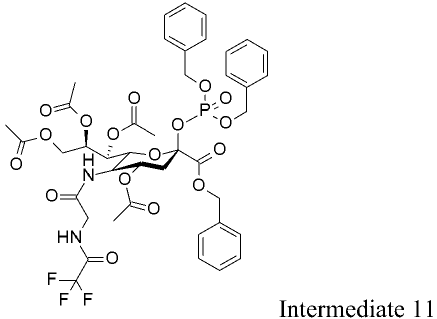
l. Debenzylation of intermediate 11 to obtain intermediate 12 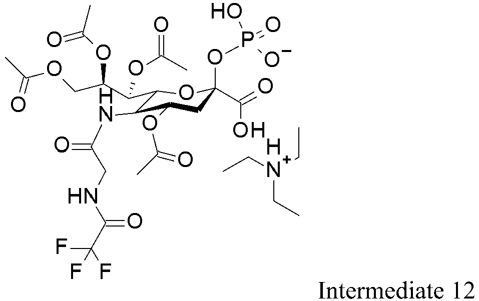
m. Triacetyl-Cytidine coupling intermediate 12 to obtain intermediate 13 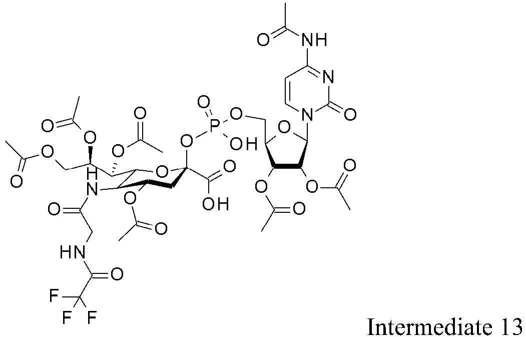
n. Acetyl and trifuoroacetamide deprotection of intermdediate 13

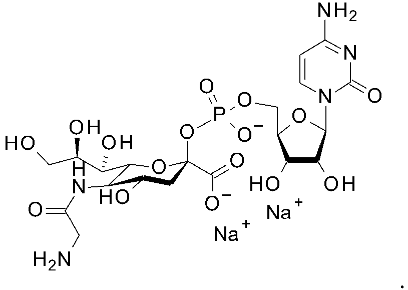

### DETAILED DESCRIPTION

It is to be understood that both the foregoing general description and the following detailed description are exemplary and explanatory only and are not restrictive of the compositions and methods of the present disclosure.

Disclosed herein is a process for the synthesis of cytidine-5'-monophospho-N-glycyl-sialic acid. IN some aspects, the synthesis comprises several steps.

In some embodiments, the process has two or more steps. In some embodiments, the process has three or more steps. In some embodiments, the process has four or more steps. In some embodiments, the process has five or more steps. In some embodiments, the process has six or more steps. In some embodiments, the process has seven or more steps. In some embodiments, the process has eight or more steps. In some embodiments, the process has nine or more steps. In some embodiments, the process has ten or more steps. In some embodiments, the process has eleven or more steps. In some embodiments, the process has twelve or more steps. In some embodiments, the process has thirteen or more steps. In some embodiments, the process has fourteen or more steps. In some embodiments, the process has two to fourteen steps. In some embodiments, the process is a 14 steps process.

In some embodiments, the process comprises one or more of Benzylation, Acetylation, Thiophenol introduction, Boc protection, Deacetylation, Boc deprotection, TFA-Gly introduction, Thiophenol removal, Phosphite introduction, Debenzylation, Triacetyl-Cytidine coupling, Acetyl and trifuoroacetamide deprotection reactions.

In some embodiments, the process is a 14 steps process. In some embodiments, the 14 steps process comprises:
Step 1: Benzylation of N-Acetylneuraminic acid (NeuAc);
Step 2: Acetylation
Step 3: Thiophenol introduction
Step 4: Boc protection
Step 5: Deacetylation
Step 6: Acetylation
Step 7: Boc deprotection
Step 8: TFA-Gly introduction
Step 9: Thiophenol removal
Step 10: Phosphite introduction
Step 11: Oxydation
Step 12: Debenzylation
Step 13: Triacetyl-Cytidine coupling
Step 14: Acetyl and trifuoroacetamide deprotection

In some embodiments, one or more steps can be eliminated and/or substituted. For example, in some embodiments, the process can have 13 steps, 12 steps, 11 steps, 10 steps, 9 steps or less. In some embodiments, one or more steps can be substituted. In some embodiments, other reagents know in the art that are compatible with the overall chemistry can be substituted.

In some embodiments, the process comprises synthesizing one or more of the compounds of Table 1:

**Table 1**

| **Cpd #** | **structure** | **IUPAC name** |
|---|---|---|
| **1** | 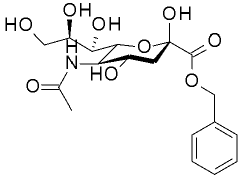 | benzyl (2S,4S,5R)-5-acetamido-2,4-dihydroxy-6-[(1R,2R)-1,2,3-trihydroxypropyl]tetrahydropyran-2-carboxylate |
| **2** | 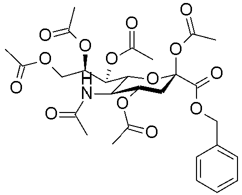 | benzyl (2R,4S,5R)-5-acetamido-2,4-diacetoxy-6-[(1S,2R)-1,2,3-triacetoxypropyl]tetrahydropyran-2-carboxylate |
| **3** | 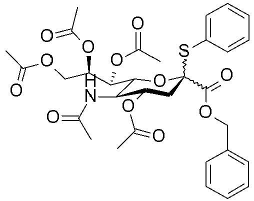 | benzyl (2R,4S,5R)-5-acetamido-4-acetoxy-2-phenylsulfanyl-6-[(1S,2R)-1,2,3-triacetoxypropyl]tetrahydropyran-2-carboxylate |
| **4** | 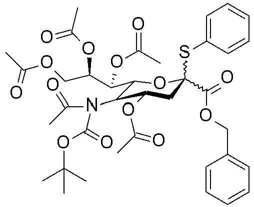 | benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate |
| **5** | 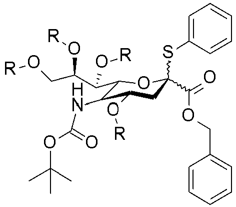 | mixture of different compounds |
| **6** | 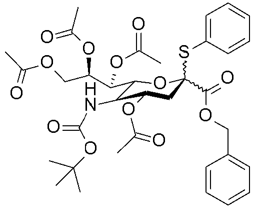 | benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate |
| **7** | 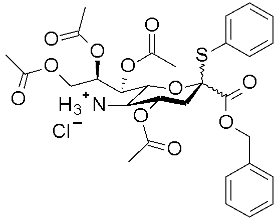 | [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium;chloride |
| **8** | 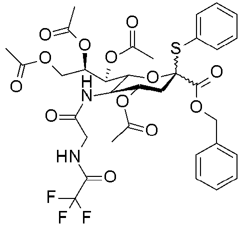 | benzyl (2R,4S,5R)-4-acetoxy-2-phenylsulfanyl-6-[(1S,2R)-1,2,3-triacetoxypropyl]-5-[[2-[(2,2,2-trifluoroacetyl)amino]acetyl]amino]tetrahydropyran -2-carboxylate |
| **9** | 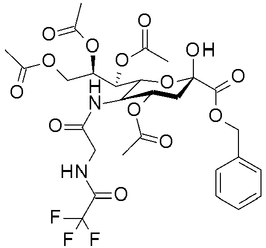 | benzyl (2S,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran -2-carboxylate |
| **10** | 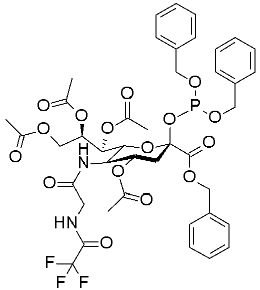 | benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran -2-carboxylate |
| **11** | 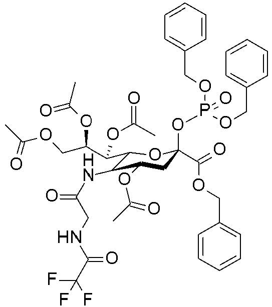 | benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran -2-carboxylate |
| **12** | 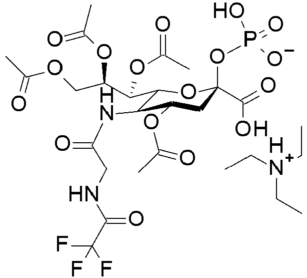 | [(2R,4S,SR)-4-acetoxy-2-carboxy-6-[(1S,2R)-1,2,3-triacetoxypropyl]-5-[[2-[(2,2,2-trifluoroacetyl)amino]acetyl]amino]tetrahydropyran -2-yl] hydrogen phosphate;triethylammonium |
| **13** | 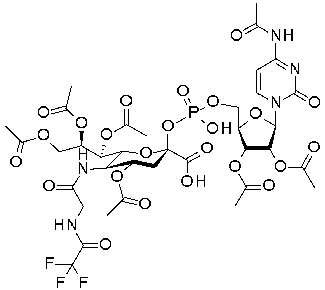 | (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran -2-carboxylic acid |
| **14** | 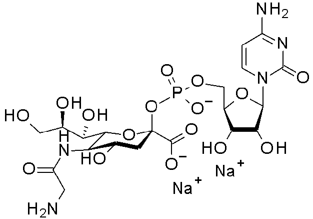 | disodium;(2R,4S,5R)-5-[(2-aminoacetyl)amino]-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-6-[(1R,2R)-1,2,3-trihydroxypropyl]tetrahydropyran-2-carboxylate |

In some embodiments, the process comprises the synthesis of the intermediates of Table 1.

In some embodiments, the starting material comprises or is N-Acetylneuraminic acid. In some embodiments, the starting material comprises or is N-Acetylneuraminic acid and the method comprises the step of synthesizing (2R,4S,5R)-5-[(2-aminoacetyl)amino]-2-[[(2R,3S,4R,SR)-5-(4-amino-2-oxo-pyrimidin-1-yl)-3,4-dihydroxy-tetrahydrofuran-2-yl]methoxy-oxido-phosphoryl]oxy-4-hydroxy-6-[(1R,2R)-1,2,3-trihydroxypropyl]tetrahydropyran-2-carboxylate or salt thereof, for example disodium;(2R,4S,5R)-5-[(2-aminoacetyl)amino]-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-6-[(1R,2R)-1,2,3-trihydroxypropyl]tetrahydropyran-2-carboxylate.

In some embodiments, the method comprises the step of synthesizing (2R,4S,5R)-5-[(2-aminoacetyl)amino]-2-[[(2R,3S,4R,5R)-5-(4-amino-2-oxo-pyrimidin-1-yl)-3,4-dihydroxy-tetrahydrofuran-2-yl]methoxy-oxido-phosphoryl]oxy-4-hydroxy-6-[(1R,2R)-1,2,3-trihydroxypropyl]tetrahydropyran-2-carboxylate or salt thereof, for example disodium;(2R,4S,SR)-5-[(2-aminoacetyl)amino]-2-[[(2R,3S,4R,SR)-5-(4-amino-2-oxo-pyrimidin-1-yl)-3,4-dihydroxy-tetrahydrofuran-2-yl]methoxy-oxido-phosphoryl]oxy-4-hydroxy-6-[(1R,2R)-1,2,3-trihydroxypropyl]tetrahydropyran-2-carboxylate.

In some embodiments, the method comprises the step of synthesizing benzyl (2S,4S,5R)-5-acetamido-2,4-dihydroxy-6-[(1R,2R)-1,2,3-trihydroxypropyl]tetrahydropyran-2-carboxylate.

In some embodiments, the method comprises the step of synthesizing benzyl (2R,4S,5R)-5-acetamido-2,4-diacetoxy-6-[(1S,2R)-1,2,3-triacetoxypropyl]tetrahydropyran-2-carboxylate.

In some embodiments, the method comprises the step of synthesizing benzyl (2R,4S,5R)-5-acetamido-4-acetoxy-2-phenylsulfanyl-6-[(1S,2R)-1,2,3-triacetoxypropyl]tetrahydropyran-2-carboxylate.

In some embodiments, the method comprises the step of synthesizing benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate.

In some embodiments, the method comprises the step of synthesizing benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate.

In some embodiments, the method comprises the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium. In some embodiments, the method comprises the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium salt. In some embodiments, the method comprises synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium;chloride

In some embodiments, the method comprises the step of synthesizing benzyl (2R,4S,5R)-4-acetoxy-2-phenylsulfanyl-6-[(1S,2R)-1,2,3-triacetoxypropyl]-5-[[2-[(2,2,2-trifluoroacetyl)amino] acetyl] amino] tetrahydropyran-2-carboxylate.

In some embodiments, the method comprises the step of synthesizing benzyl (2S,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran-2-carboxylate.

In some embodiments, the method comprises the step of synthesizing benzyl (2R,4S,5R)-4-acetoxy-2-dibenzyloxyphosphanyloxy-6-[(1S,2R)-1,2,3-triacetoxypropyl]-5-[2-[(2,2,2-trifluoroacetyl)amino]acetyl]amino]tetrahydropyran-2-carboxylate.

In some embodiments, the method comprises the step of synthesizing benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate.

In some embodiments, the method comprises the step of synthesizing [(2R,4S,5R)-4-acetoxy-2-carboxy-6-[(1S,2R)-1,2,3-triacetoxypropyl]-5-[[2-[(2,2,2-trifluoroacetyl)amino]acetyl]amino]tetrahydropyran-2-yl] hydrogen phosphate;triethylammonium.

In some embodiments, the method comprises the step of synthesizing (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid or salt thereof.

In some embodiments, the method comprises one or more steps, wherein the one or more steps comprise the step of synthesizing benzyl (2S,4S,5R)-5-acetamido-2,4-dihydroxy-6-[(1R,2R)-1,2,3-trihydroxypropyl]tetrahydropyran-2-carboxylate, the step of synthesizing benzyl (2R,4S,5R)-5-acetamido-2,4-diacetoxy-6-[(1S,2R)-1,2,3-triacetoxypropyl]tetrahydropyran-2-carboxylate, the step of synthesizing benzyl (2R,4S,5R)-5-acetamido-4-acetoxy-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate, the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium, the step of synthesizing benzyl (2R,4S,5R)-4-acetoxy-2-phenylsulfanyl-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (25,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing [(2R,4S,5R)-4-acetoxy-2-carboxy-6-[(1S,2R)-1,2,3-triacetoxypropyl]-5-[[2-[(2,2,2-trifluoroacetyl)amino]acetyl]amino]tetrahydropyran-2-yl] hydrogen phosphate;triethylammonium, the step of synthesizing (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid or salt thereof.

In some embodiments, the process is a 14 steps process as described in Example 1.

### ENUMERATED EMBODIMENTS

The disclosure also includes the following embodiments:
Embodiment I. A method of synthesizing cytidine-5'-monophospho-N-glycyl-sialic acid (GSC), the method comprising two or more steps, wherein the first step of the two or more steps comprise benzylation of N-Acetylneuraminic acid.
Embodiment II. The method of Embodiment I, wherein the first step comprises the steps of synthesizing benzyl (2S,4S,5R)-5-acetamido-2,4-dihydroxy-6-[(1R,2R)-1,2,3-trihydroxypropyl]tetrahydropyran-2-carboxylate.
Embodiment III. The method of Embodiment I, wherein the two or more steps comprises the step of synthesising (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid or a salt thereof.
Embodiment IV. The method of Embodiment I, wherein the two or more steps comprises the step of synthesizing benzyl (2S,4S,5R)-5-acetamido-2,4-dihydroxy-6-[(1R,2R)-1,2,3-trihydroxypropyl]tetrahydropyran-2-carboxylate, the step of synthesizing benzyl (2R,4S,5R)-5-acetamido-2,4-diacetoxy-6-[(1S,2R)-1,2,3-triacetoxypropyl]tetrahydropyran-2-carboxylate, the step of synthesizing benzyl (2R,4S,5R)-5-acetamido-4-acetoxy-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate, the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium, the step of synthesizing benzyl (2R,4S,5R)-4-acetoxy-2-phenylsulfanyl-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2S,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing [(2R,4S,5R)-4-acetoxy-2-carboxy-6-[(1S,2R)-1,2,3-triacetoxypropyl]-5-[[2-[(2,2,2-trifluoroacetyl)amino]acetyl]amino]tetrahydropyran-2-yl] hydrogen phosphate;triethylammonium, the step of synthesizing (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid or salt thereof
Embodiment V. A method of synthesizing cytidine-5'-monophospho-N-glycyl-sialic acid (GSC), the method comprising the step of synthesizing benzyl (2S,4S,5R)-5-acetamido-2,4-dihydroxy-6-[(1R,2R)-1,2,3-trihydroxypropyl]tetrahydropyran-2-carboxylate, the step of synthesizing benzyl (2R,4S,5R)-5-acetamido-2,4-diacetoxy-6-[(1S,2R)-1,2,3-triacetoxypropyl]tetrahydropyran-2-carboxylate, the step of synthesizing benzyl (2R,4S,5R)-5-acetamido-4-acetoxy-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate, the step of synthesizing benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate, the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium, the step of synthesizing benzyl (2R,4S,5R)-4-acetoxy-2-phenylsulfanyl-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2S,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate, the step of synthesizing [(2R,4S,5R)-4-acetoxy-2-carboxy-6-[(1S,2R)-1,2,3-triacetoxypropyl]-5-[[2-[(2,2,2-trifluoroacetyl)amino]acetyl]amino]tetrahydropyran-2-yl] hydrogen phosphate;triethylammonium, the step of synthesizing (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid or salt thereof or any combinations of the foregoing steps.
Embodiment VI. The method of Embodiment V, wherein the starting material is N-Acetylneuraminic acid.
Embodiment VII. A method of synthesizing cytidine-5'-monophospho-N-glycyl-sialic acid (GSC) from N-Acetylneuraminic acid, the method comprising one or more of benzylation, acetylation, thiophenol introduction, Boc protection, deacetylation, Boc deprotection, TFA-Gly introduction, thiophenol removal, phosphite introduction, debenzylation, triacetyl-cytidine coupling, acetyl and trifuoroacetamide deprotection reactions.
Embodiment VIII. A process for the preparation of cytidine-5'-monophospho-N-glycyl-sialic acid (GSC) comprising:
   a. Benzylation of Neu5AC to obtain intermediate 1 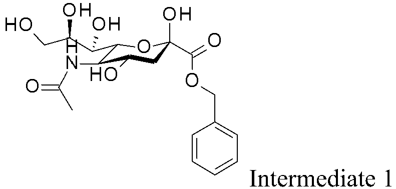
   b. Acetylation of intermediate 1 to obtain intermediate 2 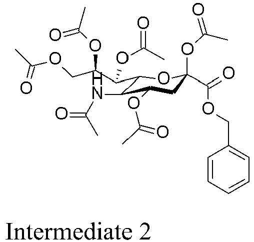
   c. Thiophenol introduction of intermediate 2 to obtain intermediate 3 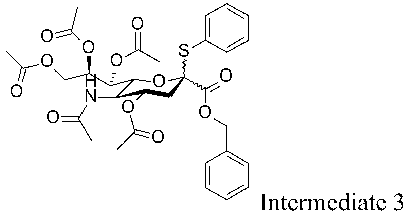
   d. Boc protection of intermediate 3 to obtain intermediate 4 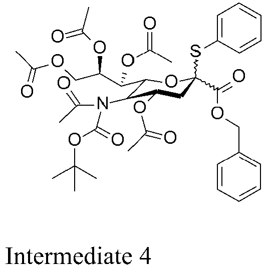
   e. Deacetylation of intermediate 4 to obtain intermediate 5 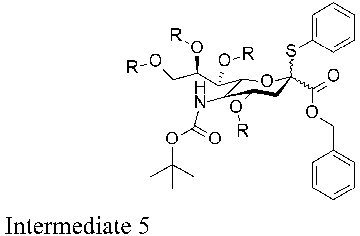
   f. Acetylation of intermediate 5 to obtain intermediate 6 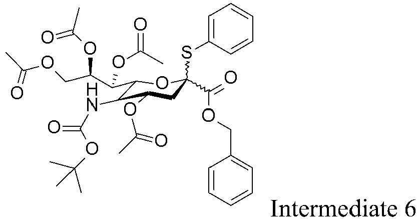
   g. Boc deprotection of intermediate 6 to obtain intermediate 7 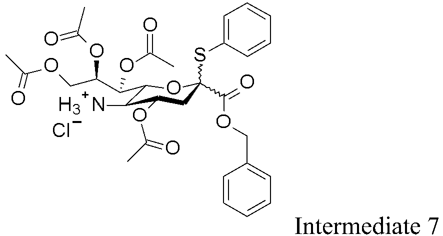
   h. TFA-Gly introduction to intermediate 7 to obtain intermediate 8 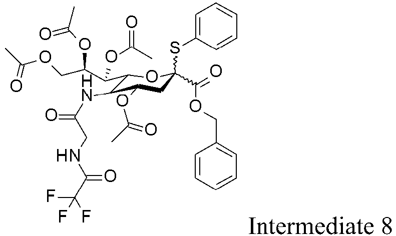
   i. Thiophenol removal to intermediate 8 to obtain intermediate 9 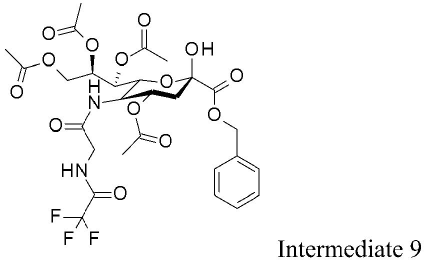
   j. Phosphite introduction to intermediate 9 to obain intermediate 10 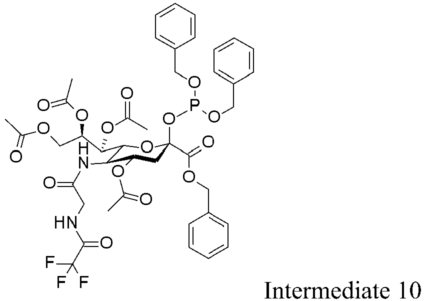
   k. oxidation of intermediate 10 to obtain intermediate 11 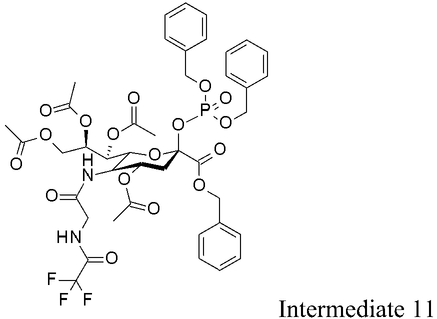
   l. Debenzylation of intermediate 11 to obtain intermediate 12 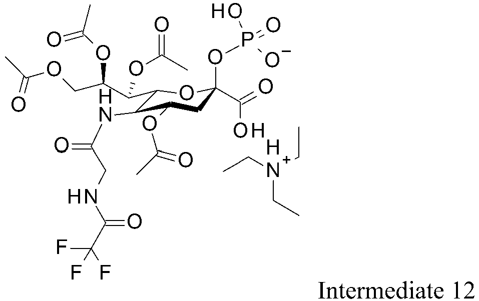
   m. Triacetyl-Cytidine coupling intermediate 12 to obtain intermediate 13 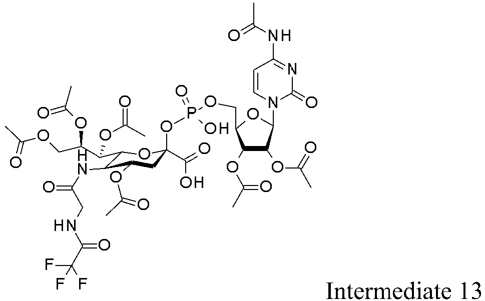
   n. Acetyl and trifuoroacetamide deprotection of intermdediate 13

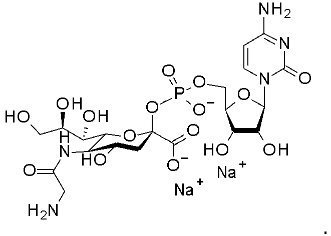

For convenience, certain terms employed in the specification, examples, and appended claims are collected here. Unless defined otherwise, all technical and scientific terms used herein have the same meaning as commonly understood by one of ordinary skill in the art to which this disclosure belongs.

As used herein, the following terms and phrases are intended to have the following meanings:

The articles "a" and "an" are used herein to refer to one or to more than one (i.e., to at least one) of the grammatical object of the article. By way of example, "an element" means one element or more than one element.

As used herein the term "comprising" or "comprises" is used in reference to compositions, methods, and respective component(s) thereof, that are present in a given embodiment, yet open to the inclusion of unspecified elements.

As used herein the term "consisting essentially of" refers to those elements required for a given embodiment. The term permits the presence of additional elements that do not materially affect the basic and novel or functional characteristic(s) of that embodiment of the disclosure.

The term "consisting of" refers to compositions, methods, and respective components thereof as described herein, which are exclusive of any element not recited in that description of the embodiment.

The term "comprising" when used in the specification includes "consisting of" and "consisting essentially of".

If it is referred to "as mentioned above" or "mentioned above", "supra" within the description it is referred to any of the disclosures made within the specification in any of the preceding pages.

If it is referred to "as mentioned herein", "described herein", "provided herein," or "as mentioned in the present text," or "stated herein" within the description it is referred to any of the disclosures made within the specification in any of the preceding or subsequent pages.

As used herein, the term "about" means acceptable variations within 20%, within 10% and within 5% of the stated value. In certain embodiments, "about" can mean a variation of +/-1%, 2%, 3%, 4%, 5%, 10% or 20%.

### EXAMPLES

### Example 1: GSC synthesis 14 steps process

### Step 1: Benzylation

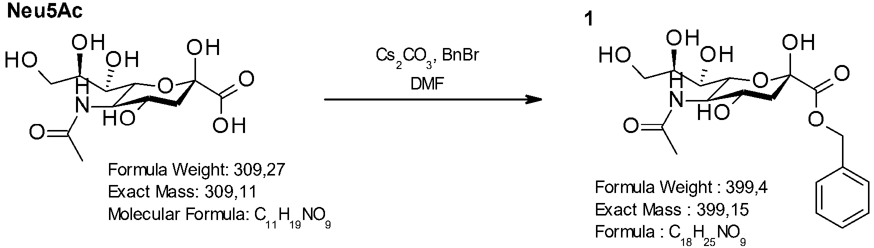

| **Entry** | **Conditions** |
|---|---|
| **Neu5Ac** | 2 L dimethylformamide |
| N-Acetylneuraminic acid | 251.4 g Cesium Carbonate |
| 400 g scale | 239 mL Benzyl Bromide |
| | 20 hours at r.t. |

### Preparation:

N-Acetylneuraminic acid (400 g, 1.288 mol, 1 eq.) was suspended in dimethylformamide (2 L). Cesium carbonate (251.4 g, 0.764 mol, 0.59 eq.) was added in three portions of 83.8 g. The resulting suspension was stirred at room temperature for 1 hour. Benzyl bromide (239 mL, 1.96 mol, 1.52 eq.) was added dropwise over a 1.5 h period time. The reaction mixture was stirred at room temperature for 16 hours.

The reaction mixture was filtered through Celite and the solid washed twice with dimethylformamide (2x300 mL). The filtrates were combined, evaporated to dryness and coevaporated once with toluene (1x800 mL) to leave an off-white gum. Isopropanol (5.2 L) was added to the residue which was then heated at 80°C for 15 minutes. The suspension was then slowly let cooled down to room temperature and stirred overnight. The precipitated solid was filtered, washed twice with isopropanol and then dried under reduced pressure at 40°C to leave 463.2 g of 1 as white solid.

### Step 2: Acetylation

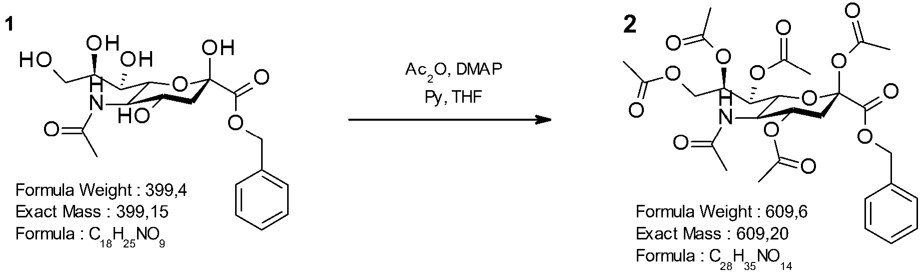

| **Entry** | **Conditions** |
|---|---|
| **1** | 4000 mL tetrahydrofuran |
| 3212-CC/1 | 1320 mL pyridine |
| 3212-DA/1 | 32.45 g 4-dimethylaminopyridine |
| 814.8 g scale | 1256 mL acetic anhydride |
| Content : 81% | addition at 0°C then 16 h at r.t. |

### Preparation:

**1** (814.8 g, 1.644 mol, 1 eq.) was suspended in tetrahydrofuran (4 L) and pyridine (1320 mL, 16.4 mol, 10 eq.). 4-dimethylaminopyridine (32.45 g, 0.263 mol, 0.16 eq.) was added at room temperature. Acetic anhydride (1.3L, 13.2 mol, 8 eq.) was added dropwise at 0°C over a 2 h period time. The reaction mixture was stirred at room temperature for 16 hours.

The reaction mixture was concentrated at 50°C until 50 mBar and ethyl acetate (10 L) was added. The resulting organic phase was washed sequentially twice with aqueous 1N HCl solution (2 x 10L), twice with saturated aqueous sodium hydrogen carbonate solution (2x10 L) and brine (3 L). The organic layer was dried over sodium sulphate, filtered and evaporated to dryness to leave 990 g of **2** as white foam.

### Step 3: Thiophenol introduction

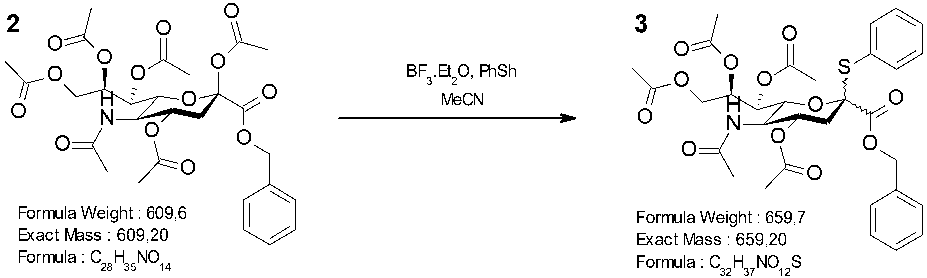

| **Entry** | **Conditions** |
|---|---|
| **2** | 2300 mL acetonitrile |
| 3212-CA/2 | 234 mL thiophenol |
| 920 g scale | 365 mL boron trifluoride diethyl etherate |
| Content : 98% | addition at 0°C then 22 h at 30°C 180 ml benzyl bromide, rt 22h |

### Preparation:

**2** (920 g, 1.479 mol, 1 eq.) was dissolved in acetonitrile (2300 mL). Thiophenol (234 mL, 2.22 mol, 1.5 eq.) was added at room temperature. Boron trifluoride diethyl etherate (365 mL, 2.957 mol, 2 eq.) was added dropwise at 0°C over a 11 minutes period time. The reaction mixture was stirred at 0°C for 5 minutes and then at 30°C for 22 hours.

Saturated aqueous sodium carbonate solution (4200 mL) was added at 0°C to the reaction mixture over a 20 minutes period time. Benzyl bromide (180 mL, 1.479 mol, 1 eq.) was added to quench the excess of thiophenol. The resulting suspension was stirred at room temperature for 22 hours.

The reaction mixture was diluted with water (4 L) and ethyl acetate (4 L). The resulting organic phase was washed with brine (2 L) and evaporated under reduced pressure to leave an orange oil.

The oil was dissolved in acetonitrile (4 L) and water (1 L). The resulting solution was washed 5 times with heptane (6x5 L), concentrated under reduced pressure and co-evaporated twice with toluene (2x4 L) and then THF (2L) to leave **3** as a white foam.

### Step 4: Boc protection

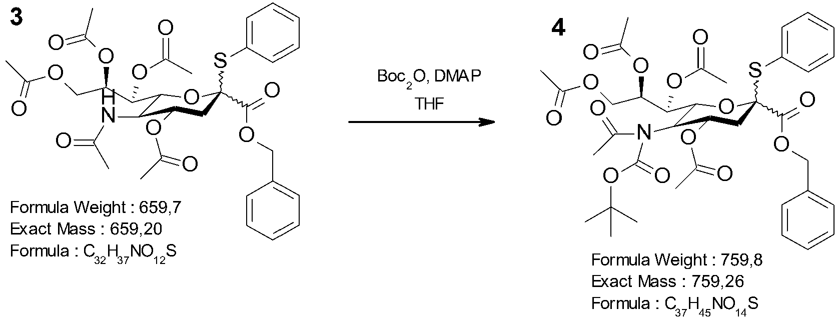

| **Entry** | **Conditions** |
|---|---|
| **3** | 2.5 L tetrahydrofuran |
| 3212-EB/3 | 557 g di-tert-butyl dicarbonate |
| 1020 g scale | 31.18 g 4-dimethylaminopyridine |
| Content : 82% | 22 h at 55°C |

### Preparation:

**3** (1020 g, 1.263 mol, 1.0 eq.) was dissolved in THF (1.5 L) and Na2SO4 (200 g) was added. The suspension was filtered and the solid was washed with THF (2 x 250 ml). To the obtained filtrate was added a solution of di-tert-butyl decarbonate (557 g, 2.526 mol, 2 eq.) in tetrahydrofuran (500 mL) and then 4-dimethylaminopyridine (31.2 g, 0.253 mol, 0.2 eq.) at room temperature. The resulting reaction mixture was stirred at 55°C for 22 hours. The reaction mixture was used directly for the next step.

### Step 5: Deacetylation

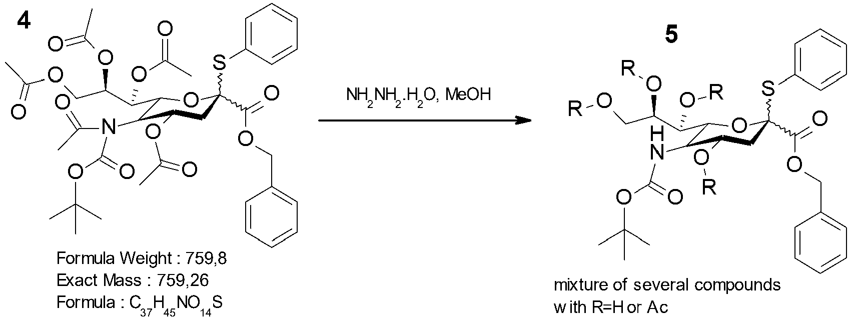

| **Entry** | **Conditions** |
|---|---|
| **4** | 2500 mL methanol |
| Reaction mixture from previous step 1.263 mol | 313 mL hydrazine monohydrate addition at 0°C then 3 h at r.t. |

### Preparation:

The reaction mixture from previous step was cooled down to 0°C and methanol (2.5L) and then hydrazine monohydrate (313 mL, 6.32 mol, 5 eq.) were added. The resulting reaction mixture was stirred at 0°C for 10 minutes and then at room temperature for 3 hours.

The reaction mixture was diluted with ethyl acetate (7.0 L) and aqueous 1N HCl solution (5 L). The organic phase was washed with aqueous 1N HCl solution (2.5 L) and then brine (2.5 L). The organic phase was evaporated under reduced pressure to leave 926 g of a dark orange oil. This crude product was directly used for the next step.

In some embodiments, hydrazine monohydrate is replaced by dimethylaminopropylamine.

### Step 6: Acetylation

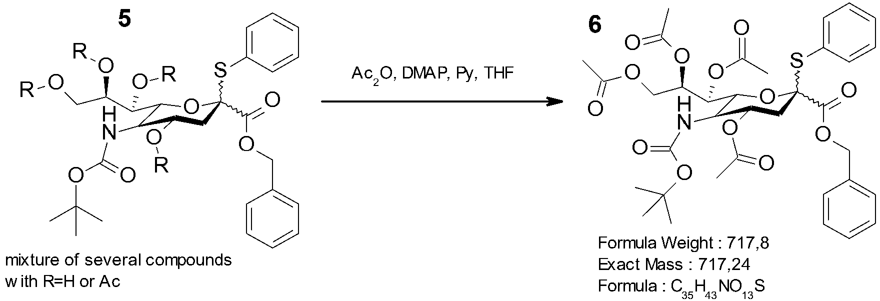

| **Entry** | **Conditions** |
|---|---|
| **5** | 2200 mL tetrahydrofuran |
| 926 g of crude product from previous step | 482 mL acetic anhydride |
| | 514 mL pyridine |
| 1.263 mol | 15.59 g 4-dimethylaminopyridine addition at 0°C then 22 h at 30°C |

### Preparation:

The crude product from previous step (926 g, 1.263 mol) was dissolved in tetrahydrofuran (2200 mL) and pyridine (514 mL, 6.316 mol, 5 eq.). Acetic anhydride (482 mL, 5.053 mol, 4 eq.) was added dropwise at 0°C over a 8 minutes period time. 4-dimethylaminopyridine (15.59 g, 0.126 mol, 0.1 eq.) was added at 0°C. The resulting reaction mixture was stirred at room temperature for 15 hours.

The reaction mixture was diluted with ethyl acetate (7 L). The resulting organic phase was washed twice with aqueous 1N HCl solution (2x7 L), twice with saturated aqueous sodium hydrogen carbonate (2 x 5 L), once with water (1 x 5L) and finally once with brine (1 x 5 L). The organic phase evaporated under reduced pressure to leave 972 g of brown oil as crude product. This crude product was purified by flash chromatography using EtOAc/heptane as eluent (gradient from 5% to 70% of ethyl acetate) to leave 740 g of **6** as a light yellow foam.

### Step 7: Boc deprotection

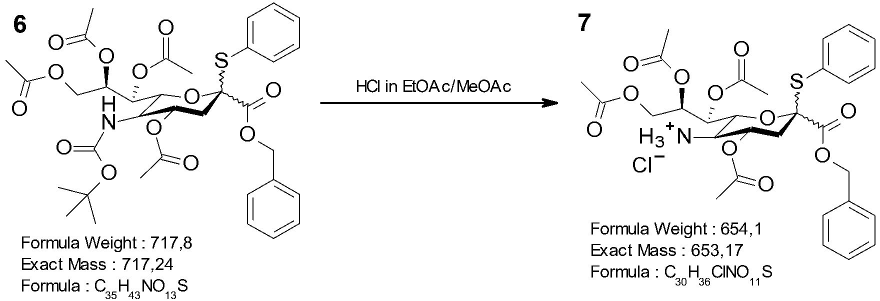

| **Entry** | **Conditions** |
|---|---|
| **6** | 1530 mL ethyl acetate |
| 3212-CC/6 | 272 ml methanol |
| 484 g scale | 534 acetyl chloride |
| Content: 96% | addition at 0°C then 2 h at r.t. |

### Preparation:

A solution of EtOAc (1050 ml) and MeOH (272 ml) was cooled down to 0°C and acetyl chloride (534 g, 6.67 mmol, 10.3 eq.) was added dropwise in 40 min. The solution was stirred fo r1 h at room temperature. The solution was then cooled down to 0°C again and a solution of **6** (484 g, 0.647 mol, 1 eq.) in ethyl acetate (480 mL) was added slowly in 20 min. The resulting reaction mixture was stirred at 0°C for 5 minutes and then at room temperature for 2 hours.

The reaction mixture was then concentrated at 30°C in a first time (HCl removal) and then at 40°C to obtain 457 g of 7 as a light yellow solid.

### Step 8: TFA-Gly introduction

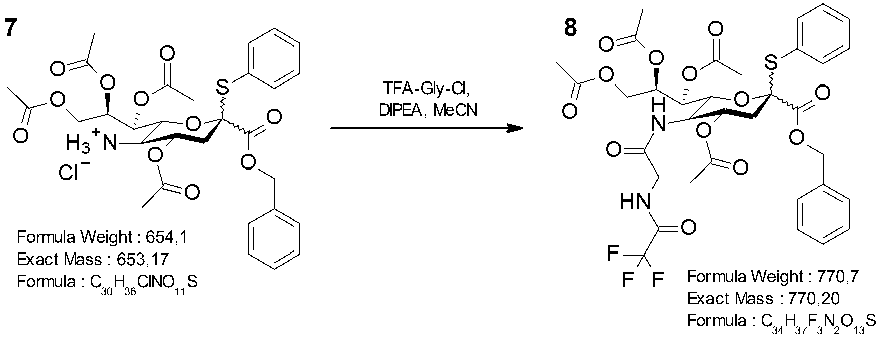

| **Entry** | **Conditions** |
|---|---|
| **7** | 2300 mL acetonitrile |
| 3212-IA/7 | 212.9 g 2-(trifluoroacetamido)acetyl chloride |
| 455 g scale | 290 mL diisopropylethylamine |
| Content: 94% | addition at 0°C then 1 h at r.t. |
| | then reacetylation |

### Preparation:

A solution of **7** (455 g, 0.652 mol, 1 eq.) in acetonitrile (1200 mL) was stirred for 10 min at room temperature. A solution of 2-(trifluoroacetamido)acetyl chloride (213 g, 0.989 mol, 1.52 eq.) in acetonitrile (1100 mL) was added and the solution was cooled down to 0°C. DIPEA (290 ml, 1.66 mol, 2.55eq.) was added dropwise in 25 min. The resulting reaction mixture was stirred at 0°C for 5 minutes and then at room temperature for 1 hour.

The reaction mixture was quenched by an addition of MeOH (52 ml, 1.30 mol, 2 eq.)

The dark orange reaction mixture was diluted with iPrOAc (2L) and HCl 1M aq. Sol. (2L). The organic phase was then washed with water (2L), NaHCO3 ½ sat..:Brine (2L:0.5L) and finally brine (1L).

The organic phase was partially concentrated under reduced pressure (half of the volume removed).

Pyridine (107 ml, 1.305 mol, 2 eq.) was added to the crude followed by acetic anhydride (95 ml, 0.979 mol, 1.5 eq.) and dimethylaminopyridine (8.2 g, 65 mmol, 0.1 eq.). The reaction mixture was stirred for 16 h at room temperature.

The dark orange reaction mixture was diluted with iPrOAc (2L) and HCl 1M aq. Sol. (2L). The organic phase was then washed with water (2L), NaHCO3 ½ sat.:Brine (2L:0.5L) and finally brine (1L).

The organic phase was concentrated under reduce pressure to obtain 569.6 g of **8** as a dark brown foam.

### Step 9: Thiophenol removal

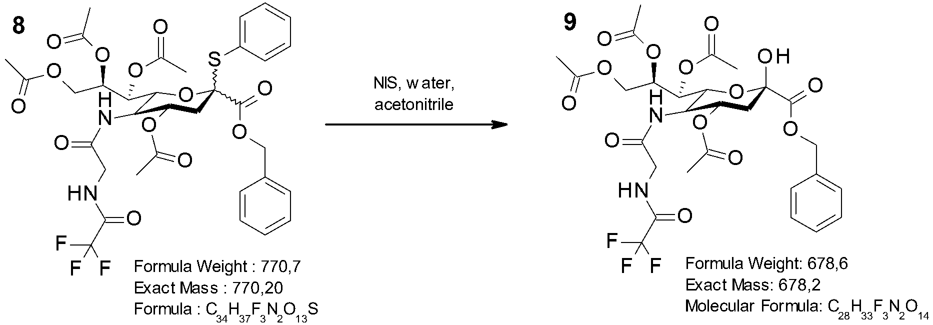

| **Entry** | **Conditions** |
|---|---|
| **8** | 3200 mL acetonitrile |
| 3212-FA/8 + | 71 mL water |
| 3212-EA/8 | 271.9 g N-Iodoosuccinimide |
| 715 g scale | 1 h at 0°C |
| Content: 85.1% | |

### Preparation:

**8** (715 g, 0.7895 mol, 1 eq.) was dissolved in acetonitrile (3200 mL) and water (71 mL, 3.947 mol, 5eq.). The reaction mixture was cooled down to 0°C and N-Iodosuccinimide (272 g, 1.184 mol, 1.5 eq.). The resulting reaction mixture was stirred at 0°C for 1 hour.

The reaction mixture was diluted with 7% sodium thiosulphate aq. sol. (3.2L). The solution was stirred for 5 min at 0°C and then diluted with ethyl acetate (3200 ml). The organic phase was washed with 7% sodium thiosulphate aq. sol. and then once with sodium bicarbonate sat. aq. (3200 ml). The organic phase was evaporated under reduced pressure at 40°C to dryness to leave 870 g of an orange gum.

The crude product was purified by flash chromatography using ethyl acetate/heptane as eluent (gradient from 20% to 100% of ethyl acetate) to leave 659 g of **9** as white foam.

### Step 10: Phosphite introduction

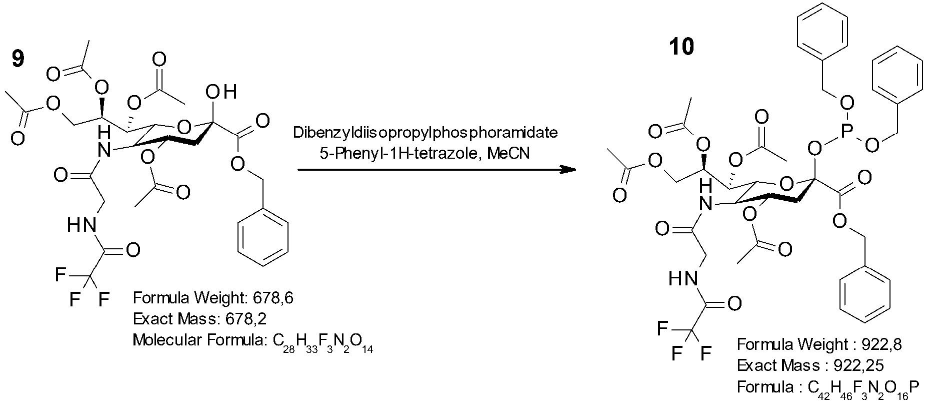

| **Entry** | **Conditions** |
|---|---|
| **9** | 1400 ml acetonitrile |
| 3212-JB/9 | 282 mL dibenzyl N,N-diisopropylphophoramidite |
| 336 g scale | 182 g 5-Phenyl-1H-Tetrazole |
| Content: 83% | 20 min at 0°C |

### Preparation:

A suspension of 5-Phenyl-1H-Tetrazole (182 g, 1.236 mol, 3 eq.) in acetonitrile (950 ml) was cooled down to 0°C. Dibenzyl N,N-diisopropylphosphoramidite (282 mL, 0.824 mol, 2 eq.) was added followed by a solution of **9** (336 g, 0.412 mol, 1 eq.) in acetonitrile (450 mL dropwise at 0°C over a 30 minutes period time. The resulting reaction mixture was stirred at 0°C for 30 minutes.

The reaction mixture was diluted with ethyl acetate (4200 mL). The resulting organic phase was washed with saturated aqueous sodium hydrogen carbonate solution (2 x 4200 mL), saturated aqueous solution of ammonium chloride (4200 mL) and then brine (4200 mL). The organic phase was dried over sodium sulphate, filtered and evaporated under reduced pressure to dryness to leave 571 g of crude product as a light orange oil.

The crude product was dissolved in ethyl acetate (500 mL). Heptane (4500 mL) was added dropwise at room temperature and the resulting suspension was stirred at room temperature for 1h. The solid was filtered, washed three times with heptane (3 x 600 ml) and dried under reduced pressure to leave 402.6 g of **10** as a light orange solid.

### Step 11: Oxydation

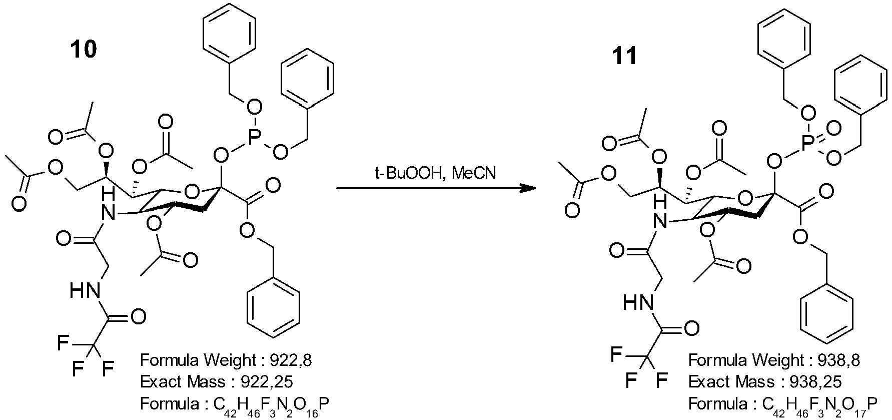

| **Entry** | **Conditions** |
|---|---|
| **10** | 1170 mL acetonitrile |
| 3212-GB/10 | 94 mL tert-butyl hydroperoxide 5.66M solution in decane addition at 0°C then 3 h at r.t. |
| 395 g scale | |
| Content: 83% | |

### Preparation:

**10** (395 g, 0.355 mol, 1 eq.) was dissolved in acetonitrile (1170 mL). Tert-butyl hydroperoxide 5.66M solution in decane (94 mL, 0.533 mmol, 1.5 eq.) was added dropwise at 0°C over a 8 minutes period time to the previous solution. The resulting reaction mixture was stirred at 0°C for 20 minutes and then at room temperature for 3 hours.

The reaction mixture was poured into ethyl acetate (280 mL). An aqueous solution of 15% by weight of sodium thiosulphate (3600 ml) and saturated aqueous solution sodium bicarbonate (15 ml) were added and the resulting mixture was stirred vigorously at room temperature for 2.5 hours. The phases were separated. The organic phase was washed with brine (3600 mL), dried over sodium sulphate, filtered and evaporated under reduced pressure to dryness to leave 432 g of crude product as yellow oil.

The crude product was suspended in isopropanol (2350 ml) and heated to 40°C until a clear solution was obtained. The solution was then cooled to room temperature and stirred overnight. The suspension was diluted with diisopropylether (1175 ml) and stirred for 5 min more at room temperature. The precipitated solid was filtered, washed twice with 5% isopropanol in diisopropylether (2 x 500 ml) and then dried under reduced pressure at 40°C to leave 280.2 g of **11** as white solid

### Step 12: Debenzylation

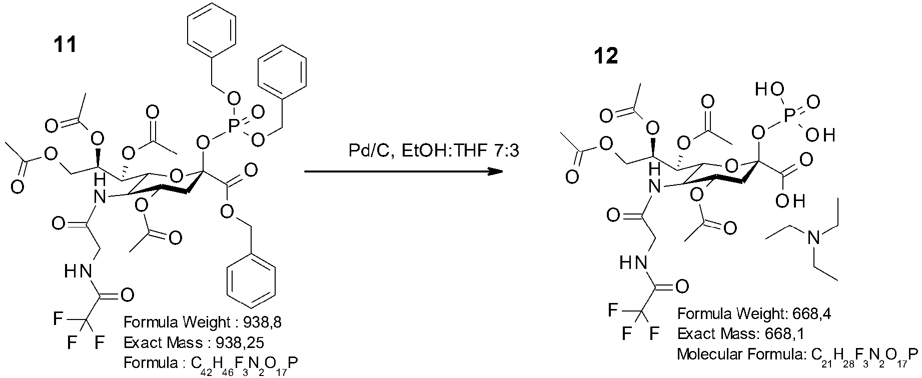

| **Entry** | **Conditions** |
|---|---|
| **11** | 300 mL tetrahydrofuran |
| 3212-EB/11 | 700 ml ethanol |
| 100 g scale | 11.7 g Pd/C (9.59% Pd) |
| Content: 99% | 7.5 mL triethylamine |
| | 4 h 30 min at r.t. |

### Preparation:

**11** (100 g, 0.1055 mol, 1 eq.) was dissolved in tetrahydrofuran (300 mL) and ethanol (700 ml) at 0°C. Pd/C (11.7 g, 10.55 mmol, 0.1 eq.) was added under an Argon atmosphere. The resulting suspension was hydrogenated at room temperature for 4.5 hours. Triethylamine (16.3 ml, 0.1160 mmol, 1.1eq.) was added after 3.5 h of reaction.

The reaction mixture was filtered through a pad of Celite. The solid was washed twice with ethanol (2x500 mL). The resulting solution was evaporated to dryness, co-evaporated once with tetrahydrofuran (500 mL) to leave 92.4 of white foam.

The crude was dissolved in tetrahydrofuran (500 ml). Diisopropylether was added dropwise over a 30 min period time followed by triethylamine (7.4 ml, 52.7 mmol, 0.5 eq.). The suspension was stirred for 1 h 15 at room temperature. The precipitated solid was filtered, washed with tetrahydrofuran:diisopropylether 1:1 (4x100 ml) and then dried under reduced pressure at 40°C to leave 77.4 g of **12** as white solid.

### Step 13: Triacetyl-Cytidine coupling

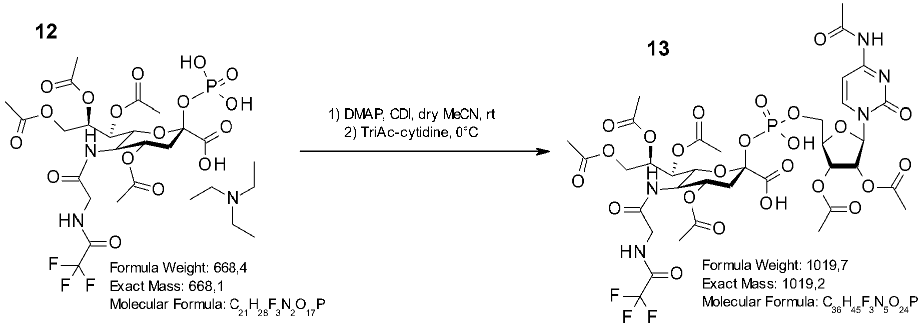

| **Entry** | **Conditions** |
|---|---|
| **13** | 490 ml dry acetonitrile |
| 3212-SB/12 | 29.3 g 3A molecular sieves |
| 97.5 g scale | 25.48 g 1,1'-Carbonyldiimidazole |
| Content: 70.4% | 68 g 2',3'-O,N⁴-triacetyl-cytidine (3212-AB/501) |
| | 25.6 g 4-Dimethylaminopyridine activation at rt then coupling at 0°C |

### Preparation:

**12** triethylamine salt (97.5 g of a compound with 70.4% content in respect of the free acids, 0.1027 mol, 1 eq.) was added to a suspension of 3A molecular sieves (29.3 g) in acetonitrile (490 ml) under an Argon atmosphere. To this solution, 4-dimethylaminopyridine (25.6 g, 0.2054 mol, 2.0 eq.) and 1,1'-Carbonyldiimidazole (23.89 g, 0.1540 mol, 1.5 eq.) were added. The reaction mixture was stirred at room temperature under Argon. After 15 min, 31P NMR from a reaction mixture aliquot showed full activation of compound **12.** The reaction mixture was cooled to 0°C. 1,1'-Carbonyldiimidazole (1.59 g, 10.27 mmol, 0.1 eq.) and 2',3'-O,N⁴-triacetyl-cytidine (68 g, 0.1643 mol, 1.6 eq.) were added to the suspension. The reaction mixture was stirred at 0°C overnight under argon and the progress of the reaction was followed by 31P NMR.

The reaction mixture was filtered through a pad of Celite and the solid was washed with dry acetonitrile (2 x 25 ml). The filtrate was kept at 0°C during the filtration. The combined cold filtrates were added dropwise to a vigorously stirred solution of isopropyl acetate (5500 mL) over a 34 minutes period time under Argon. The resulting suspension was stirred at room temperature under argon for 15 minutes. The solid was filtered under an Argon atmosphere and washed with isopropyl acetate (5x500 mL). The white solid was dried under reduced pressure at room temperature for 1.5 hour to leave 164.61 g of **13** as white solid.

### Step 14: Acetyl and trifuoroacetamide deprotection

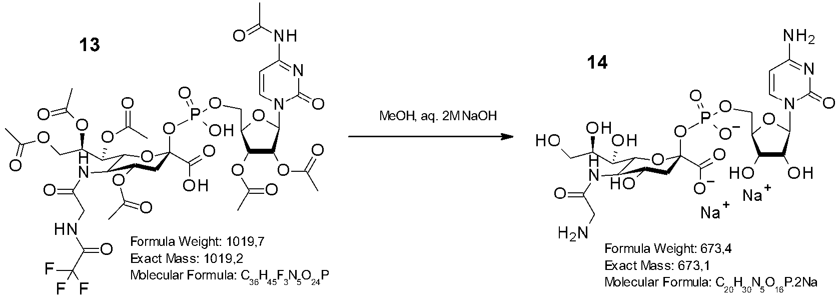

| **Entry** | **Conditions** |
|---|---|
| **13** | 375 mL methanol |
| 3212-ZMA/13 | 750 mL aqueous 2N NaOH solution |
| 164.61 g scale | |

### Preparation:

**13** (164.61 g, 0.1123 mol, 1 eq.) was dissolved in cold methanol (0°C, 375 mL). This cold solution was added dropwise to a cooled solution of aqueous 2N NaOH solution (750 mL, 13.4 eq.; internal temperature: -7°C) over a 20 minutes period time (internal temperature at the end of the addition: 5°C). The resulting solution was stirred for 1 hour at 0°C.

The reaction mixture was added dropwise to stirred ethanol (7500 mL) over a 20 min period time. The precipitated solid was filtered, washed twice with ethanol (2x500 mL) and dried under reduced pressure at 20°C for 1.5 hour to leave 81.58 g of white solid.

The white solid (81.58 g) was dissolved in aqueous 0.005N NaOH cold solution (0°C, 245 ml). The resulting solution was added dropwise to a stirred solution of methanol (1890 mL) over a 5 minutes period time. The resulting suspension was slowly cooled to -7°C (cooling ramp = - 1°C/minute) and stirred overnight at -7°C. The suspension was filtered, washed three times with cold methanol (0°C, 3 x 120 ml) and dried under high vacuum pump at room temperature for 6.5 hours to leave 73.19 g of **14** as white solid.

### Example 2

In some embodiments, the process comprises benzylation of N-Acetylneuraminic acid. In some embodiments, the process comprises the 14-step process of example 1 wherein the benzylation reaction is as follow:

### Benzylation

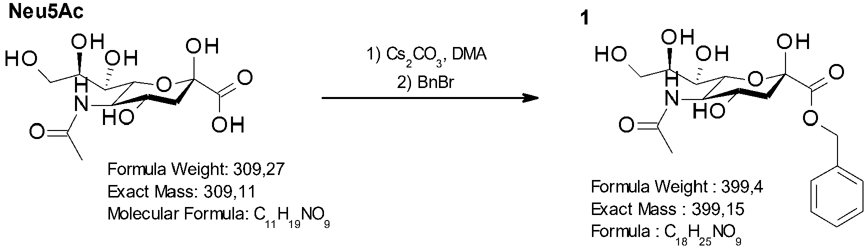

| **Entry** | **Conditions** |
|---|---|
| **Neu5Ac** | 25 mL N,N-dimethylacetamide |
| N-Acetylneuraminic acid | 3.1 g Cesium Carbonate |
| | 3 mL Benzyl Bromide |
| | Step 1: 1.25 hours at r.t. |
| 5 g scale | Step 2: 16 hours at r.t. |

**N-Acetylneuraminic acid** (5 g, 0.0161 mol, 1 eq.) was suspended in N,N-dimethylacetamide (25 mL). Cesium carbonate (3.1 g, 0.010 mol, 0.59 eq.) was added. The resulting suspension was stirred at room temperature for 1.25 hour. Benzyl bromide (3.0 mL, 0.024 mol, 1.52 eq.) was added dropwise over a 10 minutes period time. The reaction mixture was stirred at room temperature for 16 hours. The reaction mixture was used directly for the next step.

### Example 3

In some embodiments, the process comprises an acetylation step. In some embodiments, the process comprises the 14-step process of example 1 and example 2 wherein the acetylation step of compound 1 is as follow:

### Acetylation

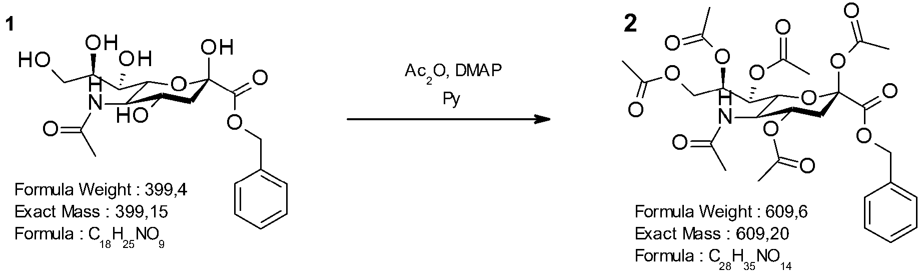

| **Entry** | **Conditions** |
|---|---|
| **1** | 13 mL pyridine |
| Reaction mixture from previous step 0.0161 mmol | 0.32 g 4-dimethylaminopyridine |
| | 12.3 mL acetic anhydride addition at 0°C then 24 h at r.t. |
| | |

### Preparation:

The reaction mixture from previous step was cooled down to 0°C. 4-dimethylaminopyridine (0.32 g, 0.003 mol, 0.16 eq.) and pyridine (13.0 mL, 0.161 mol, 10 eq.) were added. Acetic anhydride (12.3 mL, 0.129 mol, 8 eq.) was added dropwise at 0°C over a 15 minutes period time. The reaction mixture was stirred at room temperature for 24 hours.

The reaction mixture was diluted with isopropyl acetate (50 mL). The resulting organic phase was washed sequentially twice with aqueous 1N HCl solution (2 x 125 mL), twice with saturated aqueous sodium hydrogen carbonate solution (2x125 mL) and water (125 m L). The organic layer was evaporated under reduced pressure to leave 7.74 g of sticky off-white foam.

The crude product was dissolved in ethyl acetate (10 mL). Heptane (40 mL) was added dropwise at room temperature and the resulting suspension was stirred at room temperature for 1h. The solid was filtered, washed three times with heptane/EtOAc 9/1 (3 x 10 ml) and dried under reduced pressure to leave 6.59 g of **2** as white solid.

## Claims

1. A method of synthesizing cytidine-5'-monophospho-N-glycyl-sialic acid (GSC), wherein the starting material is N-acetylneuraminic acid, the method comprising:
the step of synthesizing benzyl (2S,4S,5R)-5-acetamido-2,4-dihydroxy-6-[(1R,2R)-1,2,3-trihydroxypropyl]tetrahydropyran-2-carboxylate,
the step of synthesizing benzyl (2R,4S,5R)-5-acetamido-2,4-diacetoxy-6-[(1S,2R)-1,2,3-triacetoxypropyl]tetrahydropyran-2-carboxylate,
the step of synthesizing benzyl (2R,4S,5R)-5-acetamido-4-acetoxy-2-phenylsulfanyl-6-[(1S,2R)-1,2,3-triacetoxypropyl]tetrahydropyran-2-carboxylate,
the step of synthesizing benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate,
the step of synthesizing benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate,
the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium salt,
the step of synthesizing benzyl (2R,4S,5R)-4-acetoxy-2-phenylsulfanyl-6-[(1S,2R)-1,2,3-triacetoxypropyl]-5-[[2-[(2,2,2-trifluoroacetyl)amino]acetyl]amino]tetrahydropyran-2-carboxylate,
the step of synthesizing benzyl (2S,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran-2-carboxylate,
the step of synthesizing benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran-2-carboxylate,
the step of synthesizing benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate,
the step of synthesizing [(2R,4S,5R)-4-acetoxy-2-carboxy-6-[(1S,2R)-1,2,3-triacetoxypropyl]-5-[[2-[(2,2,2-trifluoroacetyl)amino]acetyl]amino]tetrahydropyran-2-yl] hydrogen phosphate; triethylammonium, or
the step of synthesizing (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid, or a salt thereof,
or any combinations of the foregoing steps.

2. The method according to claim 1, comprising the step of synthesizing benzyl (2R,4S,5R)-4-acetoxy-2-phenylsulfanyl-6-[(1S,2R)-1,2,3-triacetoxypropyl]-5-[[2-[(2,2,2-trifluoroacetyl) amino] acetyl] amino]tetrahydropyran-2-carboxylate,
preferably wherein the step of synthesizing benzyl (2R,4S,5R)-4-acetoxy-2-phenylsulfanyl-6-[(1S,2R)-1,2,3-triacetoxypropyl]-5-[[2-[(2,2,2-trifluoroacetyl)amino]acetyl]amino]tetrahydropyran-2-carboxylate comprises the step of TFA-Gly introduction to [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium salt.

3. The method according to claim 1 or 2, comprising the step of synthesizing benzyl (2S,4S,5R)-4-acetoxy-2-hydroxy-6-[(1S,2R)-1,2,3-triacetoxypropyl]-5-[[2-[(2,2,2-trifluoroacetyl) amino] acetyl] amino]tetrahydropyran-2-carboxylate;
preferably wherein the step of synthesizing benzyl (2S,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran-2-carboxylate comprises the step of thiophenol removal from benzyl (2R,4S,5R)-4-acetoxy-2-phenylsulfanyl-6-[(1S,2R)-1,2,3-triacetoxypropyl]-5-[[2-[(2,2,2-trifluoroacetyl)amino]acetyl]amino]tetrahydropyran-2-carboxylate.

4. The method according to any one of claims 1-3, comprising the step of synthesizing benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran-2-carboxylate;
preferably wherein step of synthesizing benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran-2-carboxylate comprises the step of phosphite introduction to benzyl (2S,4S,5R)-4-acetoxy-2-hydroxy-6-[(1S,2R)-1,2,3-triacetoxypropyl]-5-[[2-[(2,2,2-trifluoroacetyl)amino]acetyl]amino]tetrahydropyran-2-carboxylate.

5. The method according to any one of claims 1-4, comprising the step of synthesizing benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate;
preferably wherein the step of synthesizing benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate comprises the step of oxidizing benzyl (2R,4S,5R)-4-acetoxy-2-dibenzyloxyphosphanyloxy-6-[(1S,2R)-1,2,3-triacetoxypropyl]-5-[[2-[(2,2,2-trifluoroacetyl)amino]acetyl]amino]tetrahydropyran-2-carboxylate.

6. The method according to any one of claims 1-5, comprising the step of synthesizing [(2R,4S,5R)-4-acetoxy-2-carboxy-6-[(1S,2R)-1,2,3-triacetoxypropyl]-5-[[2-[(2,2,2-trifluoroacetyl)amino]acetyl]amino]tetrahydropyran-2-yl] hydrogen phosphate;triethylammonium;
preferably wherein the step of synthesizing [(2R,4S,5R)-4-acetoxy-2-carboxy-6-[(1S,2R)-1,2,3-triacetoxypropyl]-5-[[2-[(2,2,2-trifluoroacetyl)amino]acetyl]amino]tetrahydropyran-2-yl] hydrogen phosphate; triethylammonium comprises the step of debenzylating benzyl (2R,4S,5R)-4-acetoxy-2-dibenzyloxyphosphoryloxy-6-[(1S,2R)-1,2,3-triacetoxypropyl]-5-[[2-[(2,2,2-trifluoroacetyl)amino]acetyl]amino]tetrahydropyran-2-carboxylate.

7. The method according to any one of claims 1-6, comprising the step of synthesizing (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid, or a salt thereof;
preferably wherein the step of synthesizing (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid, or a salt thereof comprises the step of triacetyl-cytidine coupling of [(2R,4S,5R)-4-acetoxy-2-carboxy-6-[(1S,2R)-1,2,3-triacetoxypropyl]-5-[[2-[(2,2,2-trifluoroacetyl) amino]acetyl]amino]tetrahydropyran-2-yl] hydrogen phosphate;triethylammonium.

8. The method according to any one of claims 1-7, comprising the step of acetyl and trifuoroacetamide deprotection of (2R,4S,5R)-2-[[(2R,4S,5R)-5-(4-acetamido-2-oxo-pyrimidin-1-yl)-3,4-diacetoxy-tetrahydrofuran-2-yl]methoxy-hydroxy-phosphoryl]oxy-4-acetoxy-6-[(1S,2R)-1,2,3-triacetoxypropyl]-5-[[2-[(2,2,2-trifluoroacetyl)amino]acetyl]amino]tetrahydropyran-2-carboxylic acid, or a salt thereof to synthesize GSC, or a salt thereof.

9. The method according to any one of claims 2-8, further comprising the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium salt;
preferably wherein the step of synthesizing [(3R,4S,6R)-4-acetoxy-6-benzyloxycarbonyl-6-phenylsulfanyl-2-[(1S,2R)-1,2,3-triacetoxypropyl]tetrahydropyran-3-yl]ammonium salt comprises the step of Boc deprotecting benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate.

10. The method according to any one of claims 2-9, further comprising the step of synthesizing benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate;
preferably wherein the step of synthesizing benzyl (2R,4S,5R)-4-acetoxy-5-(tert-butoxycarbonylamino)-2-phenylsulfanyl-6-[(1S,2R)-1,2,3-triacetoxypropyl]tetrahydropyran-2-carboxylate comprises the step of acetylating Intermediate 5 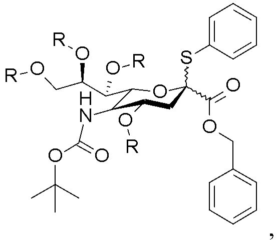 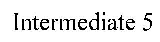 wherein each R is independently H or Ac.

11. The method according to any one of claims 2-10, further comprising the step of synthesizing 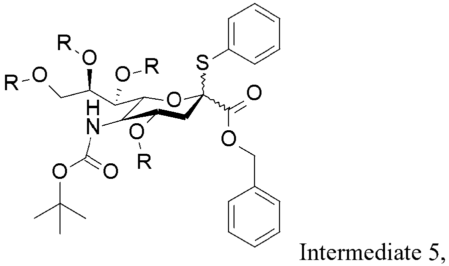 wherein each R is independently H or Ac;
preferably wherein the step of synthesizing Intermediate 5 comprises the step of deacetylating benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate.

12. The method according to any one of claims 2-11, further comprising the step of synthesizing benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate;
preferably wherein the step of synthesizing benzyl (2R,4S,5R)-4-acetoxy-5-[acetyl(tert-butoxycarbonyl)amino]-2-phenylsulfanyl-6-[(1S,2R)-1,2,3-triacetoxypropyl]tetrahydropyran-2-carboxylate comprises the step of Boc protecting benzyl (2R,4S,5R)-5-acetamido-4-acetoxy-2-phenylsulfanyl-6-[(1S,2R)-1,2,3-triacetoxypropyl]tetrahydropyran-2-carboxylate.

13. The method according to any one of claims 2-12, further comprising the step of synthesizing benzyl (2R,4S,5R)-5-acetamido-4-acetoxy-2-phenylsulfanyl-6-[(1S,2R)-1,2,3-triacetoxypropyl]tetrahydropyran-2-carboxylate;
preferably wherein the step of synthesizing benzyl (2R,4S,5R)-5-acetamido-4-acetoxy-2-phenylsulfanyl-6-[(1S,2R)-1,2,3-triacetoxypropyl]tetrahydropyran-2-carboxylate comprises the step of thiophenol introduction to benzyl (2R,4S,5R)-5-acetamido-2,4-diacetoxy-6-[(1S,2R)-1,2,3-triacetoxypropyl]tetrahydropyran-2-carboxylate.

14. The method according to any one of claims 2-13, further comprising the step of synthesizing benzyl (2R,4S,5R)-5-acetamido-2,4-diacetoxy-6-[(1S,2R)-1,2,3-triacetoxypropyl]tetrahydropyran-2-carboxylate;
preferably wherein the step of synthesizing benzyl (2R,4S,5R)-5-acetamido-2,4-diacetoxy-6-[(1S,2R)-1,2,3-triacetoxypropyl]tetrahydropyran-2-carboxylate comprises the step of acetylating benzyl (2S,4S,5R)-5-acetamido-2,4-dihydroxy-6-[(1R,2R)-1,2,3-trihydroxypropyl]tetrahydropyran-2-carboxylate.

15. The method according to any one of claims 1-14, further comprising the step of synthesizing benzyl (25,4S,5R)-5-acetamido-2,4-dihydroxy-6-[(1R,2R)-1,2,3-trihydroxypropyl]tetrahydropyran-2-carboxylate;
preferably wherein the step of synthesizing benzyl (2S,4S,5R)-5-acetamido-2,4-dihydroxy-6-[(1R,2R)-1,2,3-trihydroxypropyl]tetrahydropyran-2-carboxylate comprising the step of benzylating 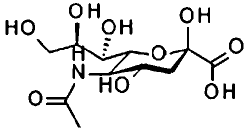 *N*-acetylneuraminic acid (Neu5Ac) or a salt thereof.

16. The method according to any one of claims 1-15, comprising
a. Benzylation of N-Acetylneuraminic acid (Neu5Ac) to obtain intermediate 1 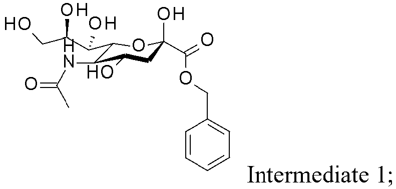
b. Acetylation of intermediate 1 to obtain intermediate 2 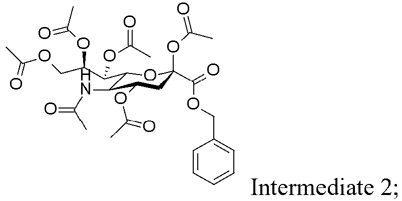
c. Thiophenol introduction of intermediate 2 to obtain intermediate 3 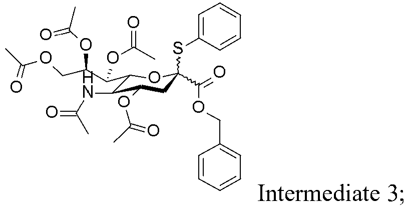
d. Boc protection of intermediate 3 to obtain intermediate 4 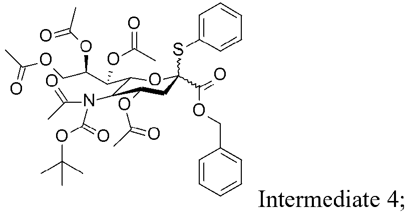
e. Deacetylation of intermediate 4 to obtain intermediate 5 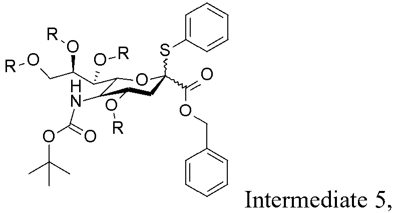 wherein each R is independently H or Ac;
f. Acetylation of intermediate 5 to obtain intermediate 6 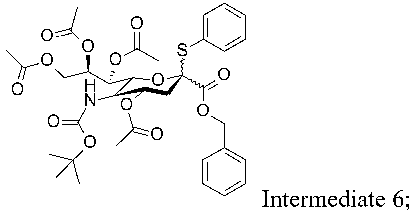
g. Boc deprotection of intermediate 6 to obtain intermediate 7 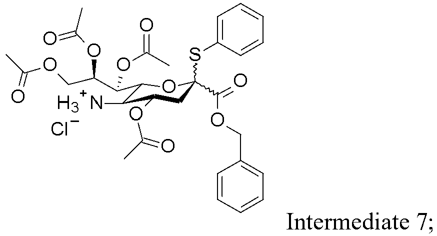
h. TFA-Gly introduction to intermediate 7 to obtain intermediate 8 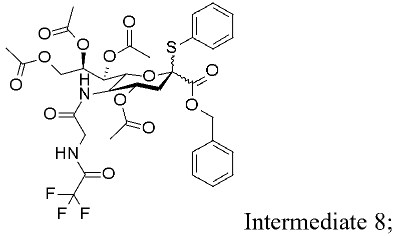
i. Thiophenol removal to intermediate 8 to obtain intermediate 9 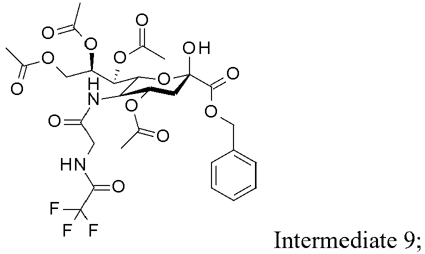
j. Phosphite introduction to intermediate 9 to obain intermediate 10 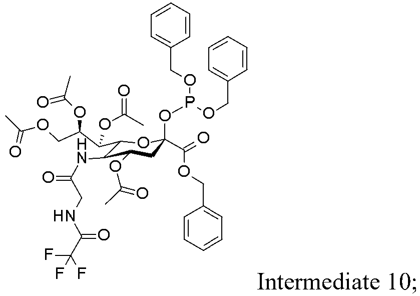
k. oxidation of intermediate 10 to obtain intermediate 11 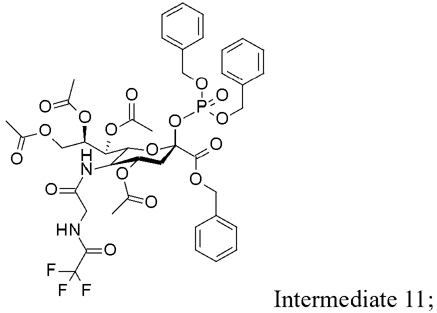
l. Debenzylation of intermediate 11 to obtain intermediate 12 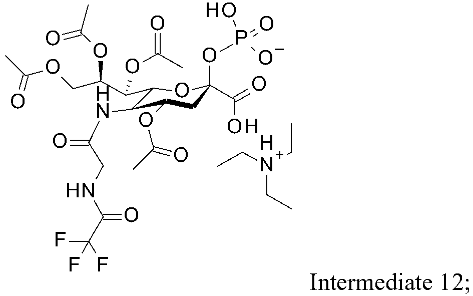
m. Triacetyl-Cytidine coupling intermediate 12 to obtain intermediate 13 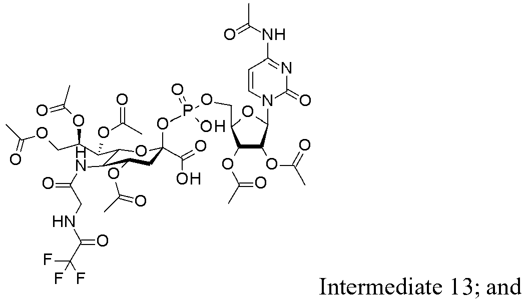
n. Acetyl and trifuoroacetamide deprotection of intermdediate 13 to obtain N-glycyl-sialic acid
